(19) Europäisches Patentamt
European Patent Office
Office européen des brevets

(11) EP 0 766 958 B1

(12) **FASCICULE DE BREVET EUROPEEN**

(45) Date de publication et mention de la délivrance du brevet: **19.11.1997 Bulletin 1997/47**

(51) Int. Cl.$^6$: **A61K 7/13**

(21) Numéro de dépôt: **96402022.6**

(22) Date de dépôt: **24.09.1996**

(54) **Composition pour la teinture d'oxydation des fibres kératiniques comprenant de la 2-amino 3-hydroxy pyridine et une base d'oxydation, et procédé de teinture**

Oxidationsfärbezusammensetzung für keratinische Fasern, die 2-Amino-3-Hydroxypyridin und ein Oxidierungsmittel enthält, und Färbeverfahren

Oxidation dyeing composition for keratinous fibers comprising 2-amino-3-hydroxypyridine and an oxidation base and dyeing process

(84) Etats contractants désignés:
**AT BE CH DE ES FR GB IT LI NL SE**

(30) Priorité: **06.10.1995 FR 9511808**

(43) Date de publication de la demande:
**09.04.1997 Bulletin 1997/15**

(73) Titulaire: **L'OREAL**
**75008 Paris (FR)**

(72) Inventeur:
**Audousset, Marie-Pascale**
**92600 Asnieres (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 559 217**
**WO-A-94/27564**
**DE-A- 2 714 831**
**DE-C- 2 739 227**
**FR-A- 2 156 527**
**GB-A- 2 180 215**

EP 0 766 958 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.23/3.4

## Description

La présente invention a pour objet une composition pour la teinture d'oxydation des fibres kératiniques, en particulier des fibres kératiniques humaines telles que les cheveux, comprenant de la 2-amino 3-hydroxy pyridine à titre de coupleur, en association avec au moins une base d'oxydation convenablement sélectionnée, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrimidine, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être notamment parmi des métadiamines aromatiques, des métaaminophénols, des métadiphénols et certains composés hétérocycliques tels que des dérivés de pyridine.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet allemand DE 2 714 831 et DE 2 739 227, des compositions pour la teinture d'oxydation des fibres kératiniques contenant de la 2-amino 3-hydroxy pyridine à titre de coupleur, en association avec des bases d'oxydation hétérocycliques, en particulier la 2,4,5,6-tetraamino pyrimidine ou avec des composés diazo comme la N-phénylamino aniline. De telles compositions ne sont cependant pas entièrement satisfaisantes notamment du point de vue de la tenue des colorations obtenues vis à vis des diverses agressions que peuvent subir les cheveux et en particulier vis à vis des shampooings.

Or, la demanderesse vient maintenant de découvrir qu'il est possible d'obtenir de nouvelles teintures peu sélectives et particulièrement résistantes, capables d'engendrer des colorations intenses dans des nuances variées, en associant de la 2-amino 3-hydroxy pyridine et au moins une base d'oxydation convenablement sélectionnée.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :

- de la 2-amino 3-hydroxy pyridine et/ou au moins l'un de ses sels d'addition avec un acide, à titre de coupleur,
- et au moins une base d'oxydation choisie parmi :

(a) les dérivés de paraphénylènediamine de formule (I), et leurs sels d'addition avec un acide :

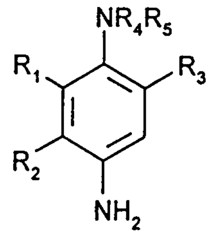

(I)

dans laquelle :

$R_1$, $R_2$ et $R_3$, identiques ou différents, représentent un atome d'hydrogène ou d'halogène, un radical alkyle en $C_1$-$C_4$, alcoxy en $C_1$-$C_4$, sulfo, carboxy, monohydroxyalkyle en $C_1$-$C_4$ ou polyhydroxyalkyle en $C_2$-$C_4$,

$R_4$ et $R_5$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), carbamylalkyle en $C_1$-$C_4$, mésylaminoalkyle en $C_1$-$C_4$, acétylaminoalkyle en $C_1$-$C_4$, uréidoalkyle en $C_1$-$C_4$, carbalcoxy($C_1$-$C_4$)aminoalkyle($C_1$-$C_4$), sulfoalkyle en $C_1$-$C_4$, pipéridinoalkyle en $C_1$-$C_4$, morpholinoalkyle en $C_1$-$C_4$, ou bien encore $R_4$ et $R_5$ forment, conjointement avec l'atome d'azote auquel ils sont liés, un hétérocycle pipéridino ou morpholino ; étant entendu que :

- au moins un des radicaux $R_1$, $R_2$, $R_3$, $R_4$ et $R_5$ est différent d'un atome d'hydrogène,
- lorsque les radicaux $R_4$ et $R_5$ représentent simultanément un atome d'hydrogène et que deux des radicaux $R_1$, $R_2$ et $R_3$ désignent simultanément un atome d'hydrogène, alors le radical $R_1$, $R_2$ ou $R_3$ restant ne désignant pas un atome d'hydrogène, est différent d'un radical méthyle,
- lorsque $R_4$ et $R_5$ ne représentent pas simultanément un atome d'hydrogène, alors un au moins des radicaux $R_1$, $R_2$ et $R_3$ doit représenter un atome d'hydrogène ;

(b) les para-aminophénols, et leurs sels d'addition avec un acide.

La composition de teinture d'oxydation conforme à l'invention permet d'obtenir des colorations aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment en ce qui concerne la résistance des colorations vis à vis des shampooings.

Parmi les radicaux alkyle en $C_1$-$C_4$ et alcoxy en $C_1$-$C_4$ de la formule (I) ci-dessus, on peut citer notamment les radicaux méthyle, éthyle, propyle, méthyloxy et éthyloxy.

Parmi les dérivés de paraphénylènediamine de formule (I) ci-dessus, utilisables à titre de base d'oxydation dans les compositions tinctoriales conformes à l'invention, on peut plus particulièrement citer la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la N-(β-hydroxypropyl) paraphénylènediamine, la 2-hydroxyméthyl paraphénylénediamine, la N,N-diméthyl 3-méthyl paraphénylénediamine, la N,N-(éthyl, β-hydroxyéthyl) paraphénylènediamine, la N-(β,γ-dihydroxypropyl) paraphénylènediamine, la 2-β-hydroxyéthyloxy paraphénylènediamine, et leurs sels d'addition avec un acide.

Parmi les para-aminophénols utilisables à titre de bases d'oxydation dans le cadre des compositions conformes à l'invention, on peut notamment citer les composés répondant à la formule (II) suivante, et leurs sels d'addition avec un acide :

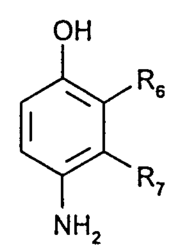

(II)

dans laquelle :

$R_6$ représente un atome d'hydrogène, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$,

$R_7$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_6$ ou $R_7$ représente un atome d'hydrogène.

Parmi les para-aminophénols de formule (II) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyethyl aminométhyl) phénol, et leurs sels d'addition avec un acide.

Les sels d'addition avec un acide utilisables dans le cadre des compositions tinctoriales de l'invention, (2-amino 3-hydroxy pyridine et bases d'oxydation), sont notamment choisis parmi les chlorhydrates, les bromhydrates, les sulfates et les tartrates.

La 2-amino 3-hydroxy pyridine et/ou le ou ses sels d'addition avec un acide, représentent de préférence de 0,0001 à 5 % en poids environ du poids total de la composition tinctoriale, et encore plus préférentiellement de 0,005 à 3 % en poids environ de ce poids.

Le ou les dérivés de paraphénylènediamine de formule (I) et/ou les para-aminophénols de formule (II) conformes à l'invention et/ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Les compositions tinctoriales conformes à l'invention peuvent contenir d'autres coupleurs différents de la 2-amino 3-hydroxy pyridine et/ou d'autres bases d'oxydation différentes des dérivés de paraphénylènediamine de formule (I) et des para-aminophénols de formule (II) et/ou des colorants directs notamment pour modifier les nuances ou les enrichir en reflets.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

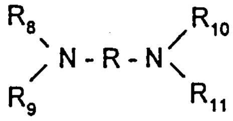 (III)

dans laquelle R est un reste propylène éventuellement substitué par un groupement hydroxyle ou un radical alkyle en $C_1$-$C_4$ ; $R_8$, $R_9$, $R_{10}$ et $R_{11}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou

substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**<u>EXEMPLES 1 et 2 COMPARATIFS</u>**

On a réalisé les compositions tinctoriales suivantes (teneurs en grammes) :

| EXEMPLE | 1 (*) | 2 |
|---|---|---|
| 2,4,5,6-tetraamino pyrimidine, monohydrate, monosulfate | 0,238 | - |
| Dichlorhydrate de 2,6-diméthyl para phénylènediamine | | 0,209 |
| 2-amino 3-hydroxy pyridine | 0,110 | 0,110 |
| Support de teinture commun | (**) | (**) |
| Eau déminéralisée q.s.p. | 100 g | 100 g |

(*) : composition ne faisant pas partie de l'invention

Il est important de noter que les compositions tinctoriales des exemples 1 et 2 contiennent la même quantité molaire de base d'oxydation, soit $1.10^{-3}$ mole.

(**) : Support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel | | |

| | | |
|---|---|---|
| de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, on a mélangé chaque composition tinctoriale avec une quantité égale en poids d'une solution d'eau oxygénée à 20 volumes (6 % en poids).

Chaque composition résultante présentait un pH d'environ 10 et a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ainsi teintes ont ensuite subi un test de résistance aux shampooing (machine Ahiba-Texomat).

Pour ce faire, on a placé les mèches de cheveux teints dans un panier que l'on a immergé dans une solution d'un shampooing standard. Le panier a été soumis à un mouvement de va-et-vient vertical de fréquence variable ainsi qu'à un mouvement de rotation qui reproduisent l'action d'un frottement manuel, ce qui a engendré la formation de mousse.

Après 3 minutes d'épreuve, on a retiré les mèches que l'on a rincé puis séché.

Les mèches ont été soumises à 6 épreuves de shampooings consécutives.

La couleur des mèches a été évaluée dans le système MUNSELL, avant et après le test de résistance aux shampooings, au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur de chaque mèche avant et après le test de résistance aux shampooings reflète la dégradation de la coloration due à l'action des shampooings et a été calculée en appliquant la formule de NICKERSON : $\Delta E = 0{,}4\ Co\Delta H + 6\Delta V + 3\ \Delta C$, telle que décrite par exemple dans "Couleur, Industrie et Technique" ; pages 14-17 ; vol. n° 5 ; 1978.

Dans cette formule, **ΔE** représente la différence de couleur entre deux mèches, **ΔH**, **ΔV** et **ΔC** représentent la variation en valeur absolue des paramètres **H**, **V** et **C**, et **Co** représente la pureté de la mèche par rapport à laquelle on désire évaluer la différence de couleur (pureté de la mèche avant le test).

Les résultats sont donnés dans le tableau II ci-dessous :

| EXEMPLE | Couleur avant le test | Couleur après le test | Dégradation de la coloration | | | |
|---|---|---|---|---|---|---|
| | | | ΔH | ΔV | ΔC | ΔE |
| | | | | | | |
| 1(*) | 0,3 Y 5,8 / 2,0 | 1,6 Y 6,0 / 1,8 | 1,3 | 0,2 | 0,2 | **2,8** |
| 2 | 4,2 YR 4,9 / 2,1 | 4,7 YR 4,9 / 1,9 | 0,5 | 0 | 0,2 | **1,0** |

(*) : exemple ne faisant pas partie de l'invention

On constate que la coloration obtenue avec la composition tinctoriale de l'exemple 2 selon l'invention, renfermant un dérivé de paraphénylènediamine de formule (I) résiste beaucoup mieux à l'action des shampooings que la coloration obtenue avec la composition tinctoriale de l'exemple 1 ne faisant pas partie de l'invention car contenant de la 2,4,5,6-tetraamino pyrimidine, composé ne répondant pas à la définition des bases d'oxydation utilisées selon l'invention et définies précédemment, et correspondant à un composé de l'art antérieur tel que décrit dans la demande de brevet allemand DE 2 714 831.

**EXEMPLES 3 et 4 COMPARATIFS**

On a réalisé les compositions tinctoriales suivantes (teneurs en grammes) :

| EXEMPLE | 3 (*) | 4 |
|---|---|---|
| | | |
| Paraphénylènediamine | 0,324 | - |
| Dichlorhydrate de 2,6-diméthyl para phénylènediamine | | 0,627 |
| | | |
| 2-amino 3-hydroxy pyridine | 0,405 | 0,405 |
| | | |
| Support de teinture commun | (**) | (**) |
| Eau déminéralisée q.s.p. | 100 g | 100 g |

(*) : composition ne faisant pas partie de l'invention
Il est important de noter que les compositions tinctoriales des exemples 3 et 4 contiennent la même quantité molaire de base d'oxydation, soit $3.10^{-3}$ mode.
(**) : Support de teinture commun :
Il est identique à celui décrit pour les exemples 1 et 2 ci-dessus.

Au moment de l'emploi, on a mélangé chaque composition tinctoriale avec une quantité égale en poids d'une solution d'eau oxygénée à 20 volumes (6 % en poids).

Chaque composition résultante présentait un pH d'environ 10 et a été appliquée pendant 30 minutes sur des mèches de cheveux gris permanentés à 90 % de blancs. Les mèches de cheveux ont ensuite été rincées, lavées avec un shampooing standard puis séchées.

Les mèches de cheveux ainsi teintes ont ensuite subi un test de résistance aux shampooing (machine Ahiba-Texomat), selon le protocole décrit pour les exemples 1 et 2 ci-dessus.

Les mèches ont été soumises à 6 épreuves de shampooings consécutives.

La couleur des mèches a été évaluée dans le système MUNSELL, avant et après le test de résistance aux shampooings, au moyen d'un colorimètre CM 2002 MINOLTA.

Les résultats sont donnés dans le tableau II ci-dessous :

| EXEMPLE | Couleur avant le test | Couleur après le test | Dégradation de la coloration | | | |
|---|---|---|---|---|---|---|
| | | | ΔH | ΔV | ΔC | ΔE |
| | | | | | | |
| 3(*) | 8,85 R 1,3 / 4,6 | 9,75 R 2,2 / 4,9 | 0,9 | 0,9 | 0,3 | **8,0** |
| 4 | 8,8 R 1,7 / 3,8 | 8,7 R 2,0 / 3,8 | 0,1 | 0,3 | 0 | **2,0** |

(*) : exemple ne faisant pas partie de l'invention

On constate que la coloration obtenue avec la composition tinctoriale de l'exemple 4 selon l'invention, renfermant un dérivé de paraphénylènediamine de formule (I) résiste beaucoup mieux à l'action des shampooings que la coloration obtenue avec la composition tinctoriale de l'exemple 3 ne faisant pas partie de l'invention car contenant de la paraphénylènediamine, composé ne répondant pas à la formule (I) des dérivés de paraphénylènediamine pouvant être utilisés selon l'invention et définie précédemment.

## Revendications

**1.** Composition pour la teinture d'oxydation des fibres kératiniques et en particulier des fibres kératiniques humaines telles que les cheveux, caractérisée par le fait qu'elle comprend, dans un milieu approprié pour la teinture :

- de la 2-amino 3-hydroxy pyridine et/ou au moins l'un de ses sels d'addition avec un acide, à titre de coupleur,
- et au moins une base d'oxydation choisie parmi :

(a) les dérivés de paraphénylènediamine de formule (I), et leurs sels d'addition avec un acide :

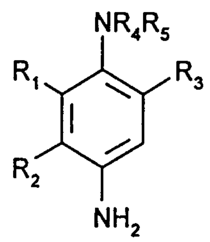

(I)

dans laquelle :

$R_1$, $R_2$ et $R_3$, identiques ou différents, représentent un atome d'hydrogène ou d'halogène, un radical alkyle en $C_1$-$C_4$, alcoxy en $C_1$-$C_4$, sulfo, carboxy, monohydroxyalkyle en $C_1$-$C_4$ ou polyhydroxyalkyle en $C_2$-$C_4$,
$R_4$ et $R_5$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), carbamylalkyle en $C_1$-$C_4$, mésylaminoalkyle en $C_1$-$C_4$, acétylaminoalkyle en $C_1$-$C_4$, uréidoalkyle en $C_1$-$C_4$, carbalcoxy($C_1$-$C_4$)aminoalkyle($C_1$-$C_4$), sulfoalkyle en $C_1$-$C_4$, pipéridinoalkyle en $C_1$-$C_4$, morpholinoalkyle en $C_1$-$C_4$, ou bien encore $R_4$ et $R_5$ forment, conjointement avec l'atome d'azote auquel ils sont liés, un hétérocycle pipéridino ou morpholino ;

étant entendu que :

- au moins un des radicaux $R_1$, $R_2$, $R_3$, $R_4$ et $R_5$ est différent d'un atome d'hydrogène,
- lorsque les radicaux $R_4$ et $R_5$ représentent simultanément un atome d'hydrogène et que deux des radicaux $R_1$, $R_2$ et $R_3$ désignent simultanément un atome d'hydrogène, alors le radical $R_1$, $R_2$ ou $R_3$ restant, ne désignant pas un atome d'hydrogène, est différent d'un radical méthyle,

- lorsque $R_4$ et $R_5$ ne représentent pas simultanément un atome d'hydrogène, alors un au moins des radicaux $R_1$, $R_2$ et $R_3$ doit représenter un atome d'hydrogène ;

(b) les para-aminophénols, et leurs sels d'addition avec un acide.

2. Composition selon la revendication 1, caractérisée par le fait que les dérivés de paraphénylènediamine sont choisis parmi la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la 2-β-hydroxyéthyloxy paraphénylènediamine, et leurs sels d'addition avec un acide.

3. Composition selon la revendication 1, caractérisée par le fait que les para-aminophénols sont choisis parmi les composés de formule (II), et leurs sels d'addition avec un acide :

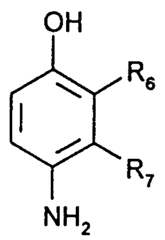

(II)

dans laquelle :

$R_6$ représente un atome d'hydrogène, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$,
$R_7$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_6$ ou $R_7$ représente un atome d'hydrogène.

4. Composition selon la revendication 3, caractérisée par le fait que les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

5. Composition selon l'une quelconque des revendications précédentes, caractérisée par le fait que les sels d'addition avec un acide sont choisis parmi les chlorhydrates, les bromhydrates, les sulfates et les tartrates.

6. Composition selon l'une quelconque des revendications précédentes, caractérisée par le fait que la 2-amino 3-hydroxy pyridine et/ou le ou ses sels d'addition avec un acide, représentent de 0,0001 à 5 % en poids du poids total de la composition tinctoriale.

7. Composition selon la revendication 6, caractérisée par le fait que la 2-amino 3-hydroxy pyridine et/ou le ou ses sels

d'addition avec un acide, représentent de 0,005 à 3 % en poids du poids total de la composition tinctoriale.

**8.** Composition selon l'une quelconque des revendications précédentes, caractérisée par le fait que le ou les dérivés de paraphénylènediamine de formule (I) et/ou le ou les para-aminophénols de formule (II) et/ou leur sels d'addition avec un acide représentent de 0,0005 à 12 % en poids du poids total de la composition tinctoriale.

**9.** Composition selon la revendication 8, caractérisée par le fait que le ou les dérivés de paraphénylènediamine de formule (I) et/ou le ou les para-aminophénols de formule (II) et/ou leur sels d'addition avec un acide représentent de 0,005 à 6 % en poids du poids total de la composition tinctoriale.

**10.** Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**11.** Composition selon l'une quelconque des revendications précédentes, caractérisée par le fait qu'elle présente un pH compris entre 3 et 12.

**12.** Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

**13.** Procédé de teinture des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 12, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**14.** Procédé selon la revendication 13, caractérisé par le fait que l'agent oxydant est choisi parmi le peroxyde d'hydrogène, le peroxyde d'urée, les bromates de métaux alcalins, les persels tels que les perborates et persulfates.

**15.** Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.

## Claims

**1.** Composition for the oxidation dyeing of keratin fibres, and in particular human keratin fibres such as the hair, characterized in that it comprises, in a medium which is suitable for dyeing:

- 2-amino-3-hydroxypyridine and/or at least one of the addition salts thereof with an acid, as coupler,
- and at least one oxidation base chosen from:

(a) the para-phenylenediamine derivatives of formula (I), and the addition salts thereof with an acid:

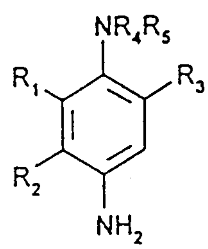
(I)

in which:

$R_1$, $R_2$ and $R_3$, which may be identical or different, represent a hydrogen or halogen atom or a $C_1$-$C_4$

alkyl, $C_1$-$C_4$ alkoxy, sulpho, carboxyl, $C_1$-$C_4$ monohydroxyalkyl or $C_2$-$C_4$ polyhydroxyalkyl radical,

$R_4$ and $R_5$, which may be identical or different, represent a hydrogen atom or a $C_1$-$C_4$ alkyl, $C_1$-$C_4$ monohydroxyalkyl, $C_2$-$C_4$ polyhydroxyalkyl, ($C_1$-$C_4$)alkoxy($C_1$-$C_4$)alkyl, carbamyl($C_1$-$C_4$)alkyl, mesylamino-($C_1$-$C_4$)alkyl, acetylamino($C_1$-$C_4$)alkyl, ureido($C_1$-$C_4$)alkyl, carb($C_1$-$C_4$)alkoxyamino($C_1$-$C_4$)alkyl, $C_1$-$C_4$ sulphoalkyl, piperidino($C_1$-$C_4$)alkyl or morpholino($C_1$-$C_4$)alkyl radical, or alternatively $R_4$ and $R_5$ form, together with the nitrogen atom to which they are attached, a piperidino or morpholino heterocycle;

it being understood that:

- at least one of the radicals $R_1$, $R_2$, $R_3$, $R_4$ and $R_5$ is other than a hydrogen atom,
- when the radicals $R_4$ and $R_5$ simultaneously represent a hydrogen atom and when two of the radicals $R_1$, $R_2$ and $R_3$ simultaneously denote a hydrogen atom, then the remaining radical $R_1$, $R_2$ or $R_3$, not denoting a hydrogen atom, is other than a methyl radical,
- when $R_4$ and $R_5$ do not simultaneously represent a hydrogen atom, then at least one of the radicals $R_1$, $R_2$ and $R_3$ must represent a hydrogen atom;

(b) para-aminophenols, and the addition salts thereof with an acid.

**2.** Composition according to Claim 1, characterized in that the para-phenylenediamine derivatives are chosen from 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-bis(β-hydroxyethyl)aniline, 4-amino-N-ethyl-N-carbamylmethylaniline, 4-amino-3-methyl-N-ethyl-N-carbamylmethylaniline, 4-amino-N-ethyl-N-(β-piperidinoethyl)aniline, 4-amino-3-methyl-N-ethyl-N-(β-piperidinoethyl)aniline, 4-amino-N-ethyl-N-(β-morpholinoethyl)aniline, 4-amino-3-methyl-N-ethyl-N-(β-morpholinoethyl)aniline, 4-amino-N-ethyl-N-(β-acetylaminoethyl)aniline, 4-amino-N-(β-methoxyethyl)aniline, 4-amino-3-methyl-N-ethyl-N-(β-acetylaminoethyl)aniline, 4-amino-N-ethyl-N-(β-mesylaminoethyl)aniline, 4-amino-3-methyl-N-ethyl-N-(β-mesylaminoethyl)aniline, 4-amino-N-ethyl-N-(β-sulphoethyl)aniline, 4-amino-3-methyl-N-ethyl-N-(β-sulphoethyl)aniline, N-[4'-(amino)phenyl]morpholine, N-[4'-(amino)phenyl]piperidine, 2-β-hydroxyethyl-para-phenylenediamine, 2-fluoro-para-phenylenediamine, 2-carboxy-para-phenylenediamine, 2-sulpho-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, and 2-β-hydroxyethyloxy-para-phenylenediamine, and the addition salts thereof with an acid.

**3.** Composition according to Claim 1, characterized in that the para-aminophenols are chosen from the compounds of formula (II), and the addition salts thereof with an acid:

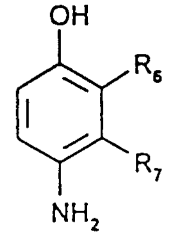

(II)

in which:

$R_6$ represents a hydrogen atom or a $C_1$-$C_4$ alkyl, $C_1$-$C_4$ monohydroxyalkyl, ($C_1$-$C_4$)alkoxy($C_1$-$C_4$)alkyl or $C_1$-$C_4$ aminoalkyl radical,

$R_7$ represents a hydrogen or fluorine atom or a $C_1$-$C_4$ alkyl, $C_1$-$C_4$ monohydroxyalkyl, $C_2$-$C_4$ polyhydroxyalkyl, $C_1$-$C_4$ aminoalkyl, cyano($C_1$-$C_4$)alkyl or ($C_1$-$C_4$)alkoxy($C_1$-$C_4$)-alkyl radical,

it being understood that at least one of the radicals $R_6$ or $R_7$ represents a hydrogen atom.

4. Composition according to Claim 3, characterized in that the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol and 4-amino-2-(β-hydroxyethylaminomethyl)phenol, and the addition salts thereof with an acid.

5. Composition according to any one of the preceding claims, characterized in that the addition salts with an acid are chosen from the hydrochlorides, hydrobromides, sulphates and tartrates.

6. Composition according to any one of the preceding claims, characterized in that the 2-amino-3-hydroxypyridine and/or the addition salt or salts thereof with an acid represent from 0.0001 to 5 % by weight relative to the total weight of the dye composition.

7. Composition according to Claim 6, characterized in that the 2-amino-3-hydroxypyridine and/or the addition salt or salts thereof with an acid represent from 0.005 to 3 % by weight relative to the total weight of the dye composition.

8. Composition according to any one of the preceding claims, characterized in that the para-phenylenediamine derivative or derivatives of formula (I) and/or the para-aminophenol(s) of formula (II) and/or the addition salts thereof with an acid represent from 0.0005 to 12 % by weight relative to the total weight of the dye composition.

9. Composition according to Claim 8, characterized in that the para-phenylenediamine derivative or derivatives of formula (I) and/or the para-aminophenol(s) of formula (II) and/or the addition salts thereof with an acid represent from 0.005 to 6 % by weight relative to the total weight of the dye composition.

10. Composition according to any one of the preceding claims, characterized in that the appropriate medium for the dyeing (or the support) consists of water or of a mixture of water and at least one organic solvent chosen from $C_1$-$C_4$ lower alkanols, glycerol, glycols and glycol ethers, aromatic alcohols, similar products and mixtures thereof.

11. Composition according to any one of the preceding claims, characterized in that it has a pH of between 3 and 12.

12. Composition according to any one of the preceding claims, characterized in that it is in the form of liquids, creams, gels or any other form which is suitable for dyeing keratin fibres, and in particular human hair.

13. Process for dyeing keratin fibres, and in particular human keratin fibres such as the hair, characterized in that at least one dye composition as defined in any one of Claims 1 to 12 is applied to these fibres, the colour being developed at acidic, neutral or alkaline pH using an oxidizing agent which is added only at the time of use to the dye composition or which is present in an oxidizing composition that is applied simultaneously or sequentially in a separate manner.

14. Process according to Claim 13, characterized in that the oxidizing agent is chosen from hydrogen peroxide, urea peroxide, alkali metal bromates, and persalts such as perborates and persulphates.

15. Multi-compartment device or multi-compartment dyeing "kit", a first compartment of which includes a dye composition as defined in any one of Claims 1 to 12 and a second compartment of which includes an oxidizing composition.

**Patentansprüche**

1. Zusammensetzung zum oxidativen Färben von Keratinfasern und insbesondere menschlichen Keratinfasern, wie dem Haar,
dadurch gekennzeichnet, daß
sie in einem zum Färben geeigneten Medium enthält:

- 2-Amino-3-hydroxypyridin und/oder mindestens eines der Additionssalze dieser Verbindung mit einer Säure als Kuppler, und

- mindestens eine Oxidationsbase, die ausgewählt ist unter:

  (a) den p-Phenylendiaminderivaten der Formel (I) und den Additionssalzen dieser Verbindungen mit einer Säure:

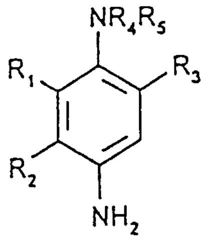

(I)

worin bedeuten:

$R_1$, $R_2$ und $R_3$, die identisch oder voneinander verschieden sind, Wasserstoff, Halogen, $C_{1-4}$-Alkyl, $C_{1-4}$-Alkoxy, Sulfo, Carboxy, $C_{1-4}$-Monohydroxyalkyl oder $C_{2-4}$-Polyhydroxyalkyl,
$R_4$ und $R_5$, die identisch oder voneinander verschieden sind, Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-Alkyl, $C_{1-4}$-Carbamoylalkyl, $C_{1-4}$-Mesylaminoalkyl, $C_{1-4}$-Acetylaminoalkyl, $C_{1-4}$-Ureidoalkyl, $C_{1-4}$Carbalkoxy-$C_{1-4}$-Aminoalkyl, $C_{1-4}$-Sulfoalkyl, $C_{1-4}$-Piperidinoalkyl, $C_{1-4}$-Morpholinoalkyl, oder $R_4$ und $R_5$ bilden gemeinsam mit dem Stickstoffatom, an das sie gebunden sind, einen Piperidino- oder Morpholino-Heterocyclus;

mit der Maßgabe, daß:

- mindestens eine der Gruppen $R_1$, $R_2$, $R_3$, $R_4$ und $R_5$ von Wasserstoff verschieden ist,
- wenn die Gruppen $R_4$ und $R_5$ gleichzeitig Wasserstoff bedeuten und zwei der Gruppen $R_1$, $R_2$ und $R_3$ gleichzeitig Wasserstoff bedeuten, dann die verbleibende Gruppe $R_1$, $R_2$ oder $R_3$, die kein Wasserstoffatom bedeutet, von Methyl verschieden ist,
- wenn $R_4$ und $R_5$ nicht gleichzeitig Wasserstoff bedeuten, mindestens eine der Gruppen $R_1$, $R_2$ und $R_3$ Wasserstoff bedeuten muß;

(b) den p-Aminophenolen und den Additionssalzen dieser Verbindung mit einer Säure.

**2.** Zusammensetzung nach Anspruch 1, dadurch gekennzeichnet, daß die p-Phenylendiaminderivate ausgewählt sind unter: 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-Amino-N,N-bis(β-hydroxyethyl)-3-methylanilin, 4-Amino-3-chlor-N,N-bis(β-hydroxyethyl)-anilin, 4-Amino-N,N-(ethyl, carbamoylmethyl)-anilin, 4-Amino-3-methyl-N,N-(ethyl, carbamoylmethyl)-anilin, 4-Amino-N,N-(ethyl, β-piperidinoethyl)-anilin, 4-Amino-3-methyl-N,N-(ethyl, β-piperidinoethyl)-anilin, 4-Amino-N,N-(ethyl, β-morpholinoethyl)-anilin, 4-Amino-3-methyl-N,N-(ethyl, β-morpholinoethyl)-anilin, 4-Amino-N,N-(ethyl, β-acetylaminoethyl)-anilin, 4-Amino-N-(β-methoxyethyl)-anilin, 4-Amino-3-methyl-N,N-(ethyl, β-acetylaminoethyl)-anilin, 4-Amino-N,N-(ethyl, β-mesylaminoethyl)-anilin, 4-Amino-3-methyl-N,N-(ethyl, β-mesylaminoethyl)-anilin, 4-Amino-N,N-(ethyl, β-sulfoethyl)-anilin, 4-Amino-3-methyl-N,N,(ethyl, β-sulfoethyl)-anilin, N-(4'-Aminophenyl)-morpholin, N-(4'-Aminophenyl)-piperidin, 2-β-Hydroxyethyl-p-phenylendiamin, 2-Fluor-p-phenylendiamin, 2-Carboxy-p-phenylendiamin, 2-Sulfo-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, 2-β-Hydroxyethyloxy-p-phenylendiamin, und den Additionssalzen dieser Verbindungen mit einer Säure.

**3.** Zusammensetzung nach Anspruch 1, dadurch gekennzeichnet, daß die p-Aminophenole unter den Verbindungen der Formel (II) und den Additionssalzen dieser Verbindungen mit einer Säure ausgewählt sind:

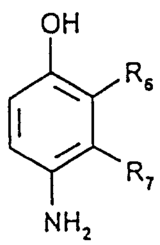

(II),

worin bedeuten:

$R_6$ Wasserstoff, $C_{1-4}$-Alkyl, $C_{1-4}$-Monohydroxyalkyl, $C_{1-4}$-Alkoxy-$C_{1-4}$-Alkyl oder $C_{1-4}$-Aminoalkyl,
$R_7$ Wasserstoff, Fluor, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-Alkyl,

mit der Maßgabe, daß mindestens eine der beiden Gruppen $R_6$ oder $R_7$ Wasserstoff bedeutet.

**4.** Zusammensetzung nach Anspruch 3, dadurch gekennzeichnet, daß die p-Aminophenole ausgewählt sind unter p-Aminophenol, 4-Amino-3-methylphenol, 4-Amino-3-fluorphenol, 4-Amino-3-hydroxymethylphenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl/aminomethyl)-phenol und den Additionssalzen dieser Verbindungen mit einer Säure.

**5.** Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

**6.** Zusammensetzung nach einem der vorhergehenden Ansprüche, dadurch gekennzeichnet, daß das 2-Amino-3-hydroxypyridin und/oder das oder die Additionssalze dieser Verbindung mit einer Säure 0,0001 bis 5 Gew.-% des Gesamtgewichts der Färbemittelzusammensetzung ausmachen.

**7.** Zusammensetzung nach Anspruch 6, dadurch gekennzeichnet, daß das 2-Amino-3-hydroxypyridin und/oder das oder die Additionssalze dieser Verbindung mit einer Säure 0,005 bis 3 Gew.-% des Gesamtgewichts der Färbemittelzusammensetzung ausmachen.

**8.** Zusammensetzung nach einem der vorhergehenden Ansprüche, dadurch gekennzeichnet, daß das oder die p-Phenylendiaminderivate der Formel (I) und/oder das oder die p-Aminophenole der Formel (II) und/oder die Additionssalze dieser Verbindungen mit einer Säure 0,0005 bis 12 Gew.-% des Gesamtgewichts der Färbemittelzusammensetzung ausmachen.

**9.** Zusammensetzung nach Anspruch 8, dadurch gekennzeichnet, daß das oder die p-Phenylendiaminderivate der Formel (I) und/oder das oder die p-Aminophenole der Formel (II) und/oder die Additionssalze dieser Verbindungen mit einer Säure 0,005 bis 6 Gew.-% des Gesamtgewichts der Färbemittelzusammensetzung ausmachen.

**10.** Zusammensetzung nach einem der vorhergehenden Ansprüche, dadurch gekennzeichnet, daß das zum Färben geeignete Medium (oder der Träger) aus Wasser oder einem Gemisch aus Wasser und mindestens einem organischen Lösungsmittel besteht, das unter den niederen $C_{1-4}$-Alkanolen, Glycerin, den Glykolen und Glykolethern, den aromatischen Alkoholen, analogen Produkten und deren Gemischen ausgewählt ist.

**11.** Zusammensetzung nach einem der vorhergehenden Ansprüche, dadurch gekennzeichnet, daß sie einen pH-Wert im Bereich von 3 bis 12 aufweist.

**12.** Zusammensetzung nach einem der vorhergehenden Ansprüche, dadurch gekennzeichnet, daß sie in Form von Flüssigkeiten, Cremes, Gelen oder beliebigen weiteren Formen vorliegt, die dazu geeignet sind, eine Färbung von Keratinfasern und insbesondere menschlichen Keratinfasern durchzuführen.

**13.** Verfahren zum Färben von Keratinfasern und insbesondere menschlichen Keratinfasern, wie dem Haar, dadurch gekennzeichnet, daß auf die Fasern mindestens eine Färbemittelzusammensetzung nach einem der Ansprüche 1 bis 12 aufgetragen und die Farbe bei einem sauren, neutralen oder alkalischen pH-Wert mit einem Oxidationsmittel

entwickelt wird, das bei der Anwendung dieser Färbemittelzusammensetzung zugegeben wird oder das in einer oxidierenden Zusammensetzung vorliegt, die gleichzeitig oder getrennt davon anschließend aufgetragen wird.

**14.** Verfahren nach Anspruch 13, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

**15.** Vorrichtung mit mehreren Abteilungen oder "Kit" zum Färben mit mehreren Abteilungen, wobei eine erste Abteilung eine Färbemittelzusammensetzung nach einem der Ansprüche 1 bis 12 und eine zweite Abteilung eine oxidierende Zusammensetzung enthält.